# EUROPEAN PATENT APPLICATION

(11) **EP 4 461 368 A1**
(43) Date of publication of application: **13.11.2024**
(21) Application number: 23305754.6
(22) Date of filing: 12.05.2023
(51) Int. Cl.: A61P 35/00, C07D 471/14, C07D 519/00, A61K 31/437, A61K 31/4375, A61K 31/4985, A61K 31/4709, A61K 31/4725

(54) **5H-PYRROLO[3,2-B:5,4-C']DIPYRIDINE DERIVATIVES AND 6,7,8,9-TETRAHYDRO-5H-PYRROLO[3,2-B:5,4-C']DIPYRIDINE DERIVATIVES USEFUL IN THE TREATMENT OF CANCER**

(71) Applicant: Institut Regional du Cancer de Montpellier, 34090 Montpellier (FR); Institut National de la Santé et de la Recherche Médicale, 75013 Paris (FR); UNIVERSITE DE MONTPELLIER, 34090 Montpellier (FR); Greenpharma, 45100 Orléans (FR)
(72) Inventor: LINARES, Laetitia, 34160 Saint Jean de Cornies (FR); CISSE, Madi, 35000 Rennes (FR); FIRMIN, Nelly, 34160 Saint Génies des Mourgues (FR); CARRERE, Sebastien, 34090 Montpellier (FR); GUILLAUMET, Gérald, 45650 Saint Jean le Blanc (FR); FRANCOIS, Benjamin, 45560 Saint Denis en Val (FR); DO, Quoc Tuan, 45100 Orléans La Source (FR); BERNARD, Philippe, 45240 La Ferté Saint-Aubin (FR)
(74) Representative: Cabinet Nony

(57) **Abstract**

The present application relates to a compound of formula (I), or any of its pharmaceutically acceptable salt: 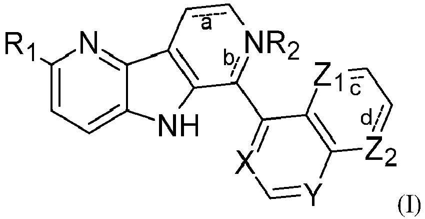 wherein R₁ represents a (C₁-C₆)alkyl group, a (C₃-C₆)cycloalkyl group, a (C₂-C₆)alkenyl group or a (C₁-C₆)alkoxy group, X and Y independently represent a -CH= group, a -CR₃= group or a -N= group, Z1 and Z2 independently represent a -CH₂- group, a =CH- group or a =N- group and R3 represents a hydrogen atom or a halogen atom.

It further relates to such a compound for its use as a medicament, and in particular for its use in treating and/or prevention of cancer, in particular cancer exhibiting recruitment of MDM2 to chromatin, more particularly selected from the group consisting of bone cancer, brain cancer, ovary cancer, breast cancer, lung cancer, colorectal cancer, osteosarcoma, skin cancer, blood cancer, including acute myeloid leukemia, pancreatic cancer, prostate cancer and liposarcoma and even more particularly selected from the group consisting of skin cancer, blood cancer, for example but not limited to acute myeloid leukemia, melanoma and liposarcoma.

## Description

This invention relates to 5H-pyrrolo[3,2-b:5,4-c']dipyridine and 6,7,8,9-tetrahydro-5H-pyrrolo[3,2-b:5,4-c']dipyridine derivatives which are efficient for treating and/or preventing cancer, in particular cancer expressing chromatin-bound MDM2 (C-MDM2 cancers) or cancers exhibiting recruitment of MDM2 to chromatin and becoming C-MDM2.

### FIELD OF THE INVENTION

Cancer refers to a group of diseases characterized by the development of abnormal cells that divide uncontrollably and can infiltrate and destroy normal body tissue. Cancer is the second-leading cause of death in the world. Numerous therapies have been developed to treat the various cancer diseases. However, sometimes, cancer cells can overcome the efficacy of the anticancer therapies. It is thus important to specifically investigate and target the mechanism of cancer and offer new and more effective therapies.

Several cancers are caused and/or enhanced by a modification in the properties of the mouse double minute 2 (MDM2) oncoprotein. MDM2 is recognized as an essential component of the tumor suppressor p53 pathway that is frequently overexpressed in its cytoplasmic form during several types of human cancers (Biderman et al., 2012, Wade et al., 2013). Recently, it has been demonstrated that MDM2 also has a p53-independent activity, i.e. may be recruited to chromatin, independently of p53, to regulate a transcriptional program implicated in amino acid metabolism and more particularly in that of serine and glycine (Riscal et al. Mol Cell. 2016 Jun 16;62(6):890-902). In other words, "chromatin-bound MDM2" or "C-MDM2" may be involved in significant modifications of cancer cell metabolism and that in a number of cancers. C-MDM2 operates independently of p53 to control serine/glycine metabolism and sustain cancer growth. Serine/glycine metabolism supports the growth of cancer cells by contributing to their anabolic demands as well as by regulating their redox state (Locasale JW. Nat Rev Cancer. 2013 Aug;13(8):572-83) and nucleotides synthesis (Cissé et al., Sci Transl Med. 2020 Jun 10;12(547)). To this day, cancer therapy has been mainly investigated using inhibitors of MDM2-p53 interaction in the cytoplasmic compartment of cancer cells to specifically interfere with MDM2 function as a negative regulator of p53. However, these p53 dependent treatments are not completely effective or induce toxicity in the treated patients. In particular, to date, most MDM2 inhibitors, that have been designated to block the MDM2-p53 binding may have low or no efficacy against advanced cancer with mutant or deficient p53.

Cancers expressing chromatin-bound MDM2 (C-MDM2) or cancers exhibiting recruitment of MDM2 to chromatin and thus becoming C-MDM2, such as but not limited to breast cancer and liposarcoma (LPS), are complex types of cancers which remain highly important to treat or prevent.

WO2023/012343 describes an Interleukin-6 (IL-6) signaling inhibitor selected from an IL-6 inhibitor, an IL-6 receptor inhibitor, an IL-6/IL-6 receptor complex inhibitor, a gp130 inhibitor and a STAT3 inhibitor, for use in a method for treating and/or preventing cancer exhibiting recruitment of MDM2 to chromatin in a patient in need thereof.

WO2016/049453 and Wang et al. Gastroenterology. "Identification of a New Class of MDM2 Inhibitor That Inhibits Growth of Orthotopic Pancreatic Tumors in Mice" 2014 October; 147(4): 893-902 both describe a pyrido[b]indole derivative, namely compound SP-141 having the following formula: 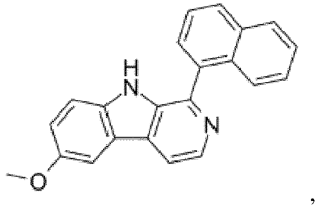 directly binding to MDM2 protein, and thus inhibiting the MDM2-p53 interaction, and therefore useful in the treatment of cancer, more particularly in the inhibition of cancer growth. Apart from said SP-141 compound, no other compound was identified to date in the literature, able to exert anticancer activity in p53 wild type, p53 mutant and p53 null cancer cells. The compound SP-141 was not developed as a candidate drug in clinical trials. It may be due to its unfavorable drug developability profile.

Therefore, there remains a need for identifying novel chemical compounds exhibiting C-MDM2 expression inhibitory activity for their use in the treatment and/or prevention of cancer, in particular cancer exhibiting recruitment of MDM2 to chromatin, and more particularly but not limited to liposarcoma, melanoma and acute myeloid leukemia. There also remains a need for identifying novel chemical compounds exhibiting C-MDM2 expression inhibitory activity independent of the p53 status, and in particular additionally minimizing host toxicity. There also remains a need for identifying novel chemical compounds exhibiting C-MDM2 expression inhibitory activity useful for reducing cancer growth and/or treating metastatic cancers. There also remains a need for identifying novel chemical compounds that selectively binds to the C-MDM2 protein and induces its autoubiquitination leading to its proteasomal degradation. There also remains a need to set up new pharmaceutical compositions and methods for treating and/or preventing cancer expressing C-MDM2, in particular but not limited to liposarcoma, melanoma and acute myeloid leukemia. There is a need to provide new methods of treatment which display a much stronger ability to induce cancer cells death than known methods, toward cancer cells expressing C-MDM2, in particular but not limited to liposarcoma, melanoma and acute myeloid leukemia cancer cells.

The present invention has for purpose to satisfy all or part of those needs.

### SUMMARY OF THE INVENTION

It has now been found that compounds as defined in formula (I) hereinafter are useful in the treatment and/or prevention of cancer, in particular cancer expressing chromatin-bound MDM2 (C-MDM2) or cancer exhibiting recruitment of MDM2 to chromatin and becoming C-MDM2, and more particularly liposarcoma, melanoma and acute myeloid leukemia.

Herein is therefore provided a compound of formula (I) as defined below.

Herein is further provided a pharmaceutical composition comprising (i) at least a compound of formula (I) as defined herein after or a pharmaceutically acceptable salt thereof, and (ii) a pharmaceutically acceptable carrier.

Herein is further provided a compound of formula (I), as defined below or a pharmaceutically acceptable salt thereof, for use as a medicament.

The present invention further relates to compounds of formula (I), as defined below, for use in the treatment and/or prevention of cancer, in particular cancer expressing chromatin-bound MDM2 (C-MDM2) or cancer exhibiting recruitment of MDM2 to chromatin, more particularly selected from the group consisting of bone cancer, brain cancer, ovary cancer, breast cancer, lung cancer, colorectal cancer, osteosarcoma, skin cancer, blood cancer, including acute myeloid leukemia, pancreatic cancer, prostate cancer and liposarcoma and even more particularly selected from the group consisting of skin cancer, liposarcoma and blood cancer, for example but not limited to liposarcoma, melanoma and acute myeloid leukemia.

The present invention further relates to a method of treatment and/or prevention of cancer, in particular cancer expressing chromatin-bound MDM2 (C-MDM2) or cancer exhibiting recruitment of MDM2 to chromatin, and more particularly liposarcoma, melanoma and acute myeloid leukemia, comprising a step of administering to a patient in need thereof a compound of formula (I) as defined below, or of a pharmaceutical composition comprising it.

The present disclosure pertains to a method for treating and/or preventing cancer in a patient in need thereof, wherein the patient has been previously classified as being affected with a cancer expressing chromatin-bound MDM2 or C-MDM2 or cancer exhibiting recruitment of MDM2 to chromatin and wherein the said method comprises a step of administering to the said patient at least a compound of formula (I) as defined herein after or a pharmaceutically acceptable salt thereof.

The present disclosure also relates to a method for treating and/or preventing cancer in a patient in need thereof, wherein the said method comprises the steps of :
a) determining eligibility of the said patient to being administered the said treatment and/or prevention by detecting recruitment of MDM2 to chromatin in a biological sample previously obtained from the said patient, and
b) administering to the said patient at least a compound of formula (I) as defined herein after or a pharmaceutically acceptable salt thereof when recruitment of MDM2 to chromatin has been detected at step a).

The present disclosure pertains to a method for treating and/or preventing cancer in a patient in need thereof, wherein the said method comprises the steps of:
a) determining eligibility of the said patient to being administered the said treatment and/or prevention by detecting recruitment of MDM2 to chromatin in a biological sample previously obtained from the said patient, and
b) administering to the said patient at least a compound of formula (I) as defined herein after or a pharmaceutically acceptable salt thereof when the said patient has been classified as being affected with a cancer exhibiting recruitment of MDM2 to chromatin at step a).

According to another embodiment, the present disclosure also relates to a pharmaceutical composition comprising (i) at least a compound of formula (I) as defined herein after or a pharmaceutically acceptable salt thereof, and (ii) a pharmaceutically acceptable carrier, for use in a method for treating and/or preventing cancer exhibiting recruitment of MDM2 to chromatin in a subject in need thereof.

According to another embodiment, the present disclosure also relates to a pharmaceutical composition comprising (i) at least a compound of formula (I) as defined herein after or a pharmaceutically acceptable salt thereof, and (ii) a pharmaceutically acceptable carrier, for use in a method for treating and/or preventing cancer in a subject in need thereof, wherein the subject has been previously classified as being affected by a cancer with recruitment of MDM2 to chromatin.

According to another embodiment, the present disclosure also relates to an *in vitro* method of determining whether a subject is affected with a cancer exhibiting a recruitment of MDM2 to chromatin, wherein said subject is intended for a therapy which comprises a compound of formula (I) according to the present invention, comprising:
- determining whether MDM2 is localized in the cancer cells nucleus of a biological sample obtained from the subject,
- wherein if MDM2 is localized in the cancer cell nucleus of the biological sample, it indicates that the subject is affected by a cancer exhibiting recruitment of MDM2 to chromatin.

### DEFINITIONS

As used herein, the term "**patient**" refers to either an animal, such as a valuable animal for breeding, company or preservation purposes, or preferably a human or a human child, which is afflicted with, or has the potential to be afflicted with, one or more diseases and conditions described herein.

In particular, as used in the present application, the term "**patient**" refers to a mammal such as a rodent, cat, dog, primate or human, preferably said patient is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "**treating**" or "**treatment**", as used herein, means reversing, alleviating, inhibiting the progress of, or preventing medical conditions of patients suffering from the diseases as described herein, in particular in the paragraph "PATHOLOGIES".

As used herein, an "**effective amount**" refers to an amount of a compound of the present invention which is effective in preventing, reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term "**effective amount**" includes "prophylaxis-effective amount" as well as "treatment-effective amount".

As used herein, the terms "**prevent**" or "**preventing**" with respect to a disease or disorder relate to prophylactic treatment of the diseases as described herein. Prevention may include, but is not limited to, preventing or delaying onset or progression of the diseases as described herein and/or maintaining one or more symptoms of the the diseases as described herein at a desired or sub-pathological level. The term "prevent" does not require the 100% elimination of the possibility or likelihood of occurrence of the event. Rather, it denotes that the likelihood of the occurrence of the event has been reduced in the presence of a composition or method as described herein. More particular, "prevent" or "preventing" refers to a decrease in the risk of occurrence of a cancer disease or symptom in a patient. As indicated above, the prevention may be complete, i.e., no detectable symptoms or disease, or partial, such that fewer symptoms or less severity of the disease are observed than would likely occur in the absence of treatment.

The term "**prophylaxis-effective amount**" refers to a concentration of compound of this invention that is effective in inhibiting, preventing, decreasing the likelihood of the diseases as described herein.

Likewise, the term "**treatment-effective amount**" refers to a concentration of compound that is effective in treating a disease as described herein.

As used herein "**pharmaceutically"** or "**pharmaceutically acceptable"** refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. By way of example of pharmaceutically acceptable carrier, sterile water, saccharides such as sucrose or saccharose, starches, sugar alcohols such as sorbitol, polymers such as PVP or PEG, lubricating agents, such as magnesium stearate, preservatives, dyeing agents or flavors can be mentioned.

The term "**MDM2**" has its general meaning in the art and refers to mouse double minute 2 oncoprotein. The term "MDM2" also refers to E3 ubiquitin-protein ligase having the UniProtKB accession number Q00987.

The term "**C-MDM2 expression inhibitor**" refers to a compound that selectively binds to the chromatin-bound MDM2 protein or C-MDM2 protein and induces its autoubiquitination and proteasomal degradation. As used herein, the term "selectively binds to the C-MDM2 protein" refers to a compound that preferentially binds to C-MDM2 protein, respectively, than to other ubiquitin-protein ligases, or related enzymes or related transporters. Compounds that block or inactivate the chromatin function of MDM2 may also down regulate enzymes related to PHGDH, PSAT, PSPH, or other members of the SLC1 family of proteins, which are C-MDM2 downstream regulated elements, and are contemplated the present invention.

In the context of the present disclosure, a "**cancer exhibiting recruitment of MDM2 to chromatin**" means that MDM2 is localized in the cell nucleus.

In the following description, the terms "**cancer expressing chromatin-bound MDM2 (C-MDM2)**" and "**cancer exhibiting recruitment of MDM2 to chromatin**" are considered as equivalent terms.

At last, the term "cancer becoming C-MDM2" means a cancer not spontaneously expressing chromatin-bound MDM2 (C-MDM2) before any treatment and that expresses C-MDM2 after treatment, for example after one or more cycles of treatments by radiotherapy, chemotherapy and/or immunotherapies.

### DETAILED DESCRIPTION OF THE INVENTION

The inventors have in particular found that the compounds of formula (I) are endowed with an activity of inhibiting expression of C-MDM2 and degradation thereof.

Herein is provided a compound of formula (I), or any of its pharmaceutically acceptable salt: 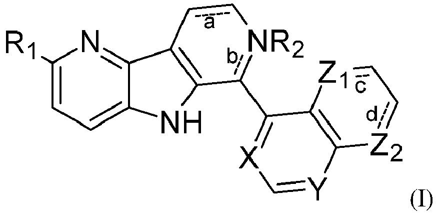 wherein:
R₁ represents a (C₁-C₆)alkyl group, a (C₃-C₆)cycloalkyl group, a (C₂-C₆)alkenyl group or a (C₁-C₆)alkoxy group, wherein one or two -CH₂- groups as present in said groups may be replaced by -O-, -S- or -NH- and wherein said groups may be substituted by one or two hydroxy group or (C₁-C₆)alkoxy group,
X and Y independently represent a -CH= group, a -CR₃= group or a -N= group,
Z1 and Z2 independently represent a -CH₂- group, a =CH- group or a =N- group,
R3 represents a hydrogen atom or a halogen atom,
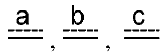 and 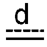 independently represent a single or double bond, and
R₂ is absent when 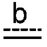 is a double bond and represents a hydrogen atom when 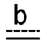 is a double bond, and wherein when 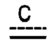 and 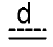 both represent a single bond, then Z1 and Z2 both represent a -CH₂- group.

As illustrated in the examples, MDM2 degradation was assessed on cancer cells exhibiting or not a recruitment of MDM2 to chromatin (IB111, presence of C-MDM2 or hPac/ZR75.1 absence of C-MDM2), after treatment with compounds of the present invention. Cell survival after treatment with compounds of the present invention was also evaluated. At last, the specific anti-tumor efficacy on liposarcoma has been evidenced through *in vivo* evaluation on mice engrafted with liposarcoma cells IB 111.

It has been furthermore observed that the compounds of formula (I) of the present invention exhibit developability properties of great interest. In particular solubility of the compounds of formula (I) according to the present invention has been assessed and considered convenient according to the pharmacopeial standards.

In the context of the present invention, the term:
- "halogen" is understood to mean chlorine, fluorine, bromine, or iodine, and in particular denotes chlorine, fluorine or bromine,
- "(C₁-C₆)alkyl" as used herein refers to a linear or branched saturated hydrocarbon-based aliphatic group, comprising, from 1 to 6 carbon atoms, in particular a (C₁-C₃)alkyl or a (C₁-C₅)alkyl. Examples are, but are not limited to, methyl, ethyl, 1-propyl, 2-propyl, butyl, pentyl,
- an "(C₂-C₆)alkene group" as used herein refers to a linear or branched unsatured or partially unsaturated aliphatic group containing conjugated or non-conjugated double bond(s), comprising from 2 to 6 carbon atoms;
- "(C₃-C₆)cycloalkyl" as used herein respectively refers to cyclic saturated hydrocarbon comprising from 3 to 6 carbon atoms. Examples are, but are not limited to cyclopropyl, cyclobutyl, cyclopentyl, cyclohexyl, and
- "(C₁-C₆)alkoxy" as used herein respectively refers to O-(C₁-C₆)alkyl moiety, wherein alkyl is as defined above. Examples are, but are not limited to, methoxy, ethoxy, 1-propoxy, 2-propoxy, butoxy.

The compounds of the invention may exist in the form of free bases or of addition salts with pharmaceutically acceptable acids.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, palmitate, pamoate, pectinate, persulfate, 3-phenylpropionate, pivalate, propionate, stearate, succinate, sulfate, tartrate, thiocyanate, p-toluenesulfonate, undecanoate, valerate salts, and the like.

Suitable physiologically acceptable acid addition salts of compounds of formula (I) include hydrobromide, tartrate, citrate, trifluoroacetate, ascorbate, hydrochloride, triflate, maleate, mesylate, formate, acetate and fumarate.

The compounds of formula (I) and or salts thereof may form solvates or hydrates and the invention includes all such solvates and hydrates.

The terms "hydrate" and "solvate" simply mean that the compounds (I) according to the invention can be in the form of a hydrate or solvate, *i.e.* combined or associated with one or more water or solvent molecules. This is only a chemical characteristic of such compounds, which can be applied for all organic compounds of this type.

The compounds of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

The compounds of formula (I) and any of compounds **(1)** to **(17)** as defined herein after may be under amorphous or crystalline forms, which are encompassed within the scope of the present invention as well.

In one embodiment, R₁ represents a (C₁-C₄)alkyl group or a (C₁-C₄)alkoxy group, wherein said groups may be substituted by one or two hydroxy group or (C₁-C₂)alkoxy group, in particular R₁ represents a (C₁-C₄)alkoxy group, and more particularly R1 represents a methoxy group.

In one embodiment,
- X, Y, Z₁ and Z₂ simultaneously represent a -CH= group with 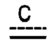 and 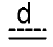 both representing a double bond,
- X and Z₂ simultaneously represent a -CH= group and Y and Z₁ represent a =N- group, with 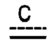 and 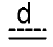 both representing a double bond,
- Y, Z₁, and Z₂ simultaneously represent a -CH= group and X represents a =N- group, with 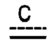 and 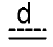 both representing a double bond,
- X, Y, and Z₂ simultaneously represent a -CH= group and Z1 represents a =N- group, with 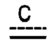 and 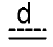 both representing a double bond,
- X, Z₁, and Z₂ simultaneously represent a -CH= group and Y represents a =N- group, with 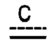 and 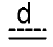 both representing a double bond,
- Y, Z₁, and Z₂ simultaneously represent a -CH= group and X represents a =N- group, with 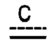 and 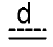 both representing a double bond,
- X, Y and Z₁ simultaneously represent a -CH= group and Z₂ represents a =N- group, with 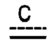 and 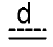 both representing a double bond, or
- X and Y simultaneously represent a -CH= group, and Z₁ and Z₂ represent a -CH₂- group,
with 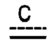 and 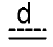 both representing a single bond.

In one embodiment, 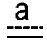 and 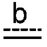 both represent a double bond and R₂ does not exist, or 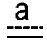 and 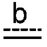 both represent a single bond and R₂ is a hydrogen atom.

In another embodiment, R₁ represents a methoxy group.

In another embodiment, the halogen atom is a fluorine atom.

In another embodiment, R₃ represents a hydrogen atom.

In another embodiment, R₃ represents a fluorine atom.

In another embodiment, 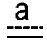 and 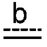 both represent a double bond, R2 is absent, X is a -CH= group and (i) 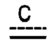 and 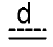 represent a double bond and Y, Z1 and Z2 are simultaneously a -CH= group or simultaneously a -N= group or (ii) 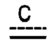 and 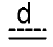 represent a single bond and Y is a -CH= group and Z1 and Z2 are simultaneously a -CH₂- group.

In another embodiment, the pharmaceutically acceptable salt of the compound according to the present invention is selected from a hydrochloride, a phosphate, an oxalate and a citrate salt.

According to a particular embodiment of the present invention, the compound of formula (I) is selected from:
- (1) 2-methoxy-6-(naphthalen-1-yl)-5H-pyrrolo[3,2-b:5,4-c']dipyridine,
- (2) 2-methoxy-6-(1,5-naphthyridin-4-yl)-5H-pyrrolo[3,2-b:5,4-c']dipyridine,
- (3) 2-methoxy-6-(pyrido[2,3-b]pyrazin-8-yl)-5H-pyrrolo[3,2-b:5,4-c'] dipyridine,
- (4) 2-methoxy-6-(naphthalen-1-yl)-5H-pyrrolo[3,2-b:5,4-c']dipyridine hydrochloride,
- (5) 2-methoxy-6-(quinoline-8-yl)-5H-pyrrolo[3,2-b:5,4-c']dipyridine,
- (6) 2-methoxy-6-(quinolin-4-yl)-5H-pyrrolo[3,2-b:5,4-c']dipyridine,
- (7) 6-(isoquinolin-1-yl)-2-methoxy-5H-pyrrolo[3,2-b:5,4-c']dipyridine,
- (8) 2-methoxy-6-(quinolin-5-yl)-5H-pyrrolo[3,2-b:5,4-c']dipyridine,
- (9) 2-methoxy-6-(naphthalen-1-yl)-6,7,8,9-tetrahydro-5H-pyrrolo[3,2-b:5,4-c'] dipyridine,
- (10) 2-methoxy-6-(naphthalen-1-yl)-5H-pyrrolo[3,2-b:5,4-c']dipyridine fumarate,
- (11) 2-methoxy-6-(naphthalen-1-yl)-5H-pyrrolo[3,2-b:5,4-c']dipyridine 2-hydroxypropane-1,2,3-tricarboxylate,
- (12) 2-methoxy-6-(naphthalen-1-yl)-5H-pyrrolo[3,2-b:5,4-c']dipyridine phosphate,
- (13) 2-methoxy-6-(naphthalen-1-yl)-5H-pyrrolo[3,2-b:5,4-c']dipyridine sulfate,
- (14) 2-methoxy-6-(5,6,7,8-tetrahydronaphthalen-1-yl)-5H-pyrrolo[3,2-b:5,4-c'] dipyridine,
- (15) 6-(4-fluoronaphthalen-1 -yl)-2-methoxy-5H-pyrrolo [3,2-b: 5,4-c'] dipyridine,
- (16) 2-methoxy-6-(5,6,7,8-tetrahydronaphthalen-1-yl)-5H-pyrrolo[3,2-b:5,4-c']dipyridine hydrochloride,
- (17) 6-(4-fluoronaphthalen-1 -yl)-2-methoxy-5H-pyrrolo [3,2-b: 5,4-c']dipyridine hydrochloride,
   and their pharmaceutically acceptable salts.

Another embodiment is a compound selected from the above list, or a pharmaceutically acceptable salt thereof, for use as a medicament, especially as a C-MDM2 expression inhibitor.

Another embodiment is a compound selected from the above list, or a pharmaceutically acceptable salt thereof, for use in the treatment of cancer, in particular cancer expressing C-MDM2, more particularly selected from the group consisting of bone cancer, brain cancer, ovary cancer, breast cancer, lung cancer, colorectal cancer, osteosarcoma, skin cancer, blood cancer, including acute myeloid leukemia, pancreatic cancer, prostate cancer and liposarcoma and even more particularly selected from the group consisting of skin cancer, liposarcoma and blood cancer, for example but not limited to liposarcoma, melanoma and acute myeloid leukemia.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

### SCHEME 1

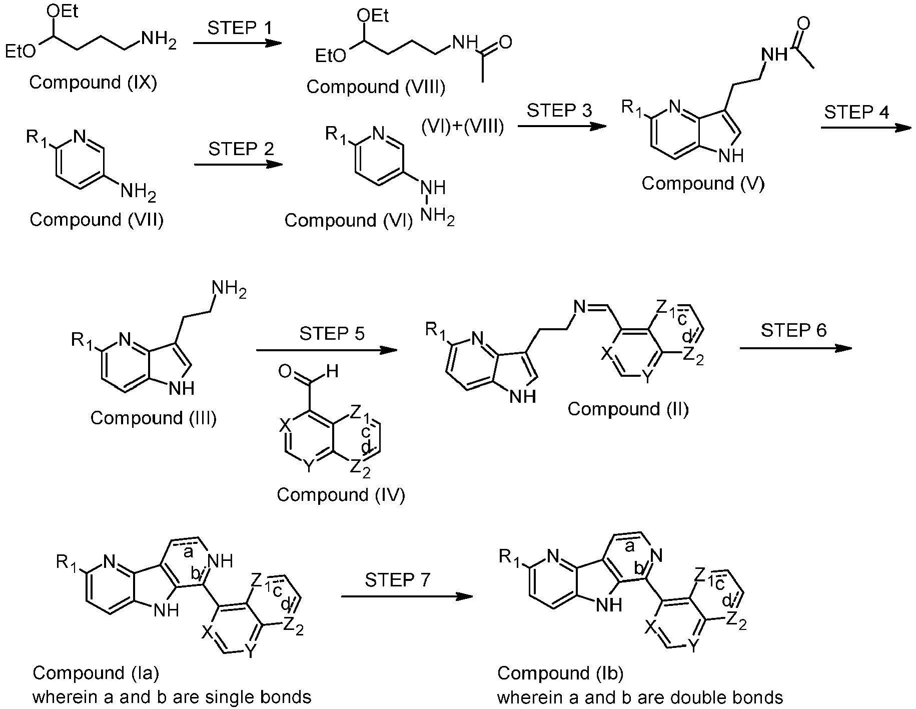

In SCHEMES 1, 2 and 3 compounds of formula (Ia) and (Ib), both encompassed within formula (I) respectively correspond to a compound of formula (Ia), wherein 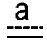 and 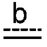 both represent single bonds while 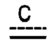 and 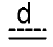 independently represent a single or double bond and to a compound of formula (Ib), wherein 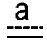 and 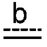 both represent double bonds while 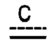 and 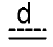 independently represent a single or double bond.

According to SCHEME 1, in which R₁, Z₁, Z₂, X , Y, 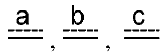 and 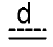 are as defined above, a compound of formula (IX) can be converted in STEP 1 to a compound of formula (VIII) under acetylation conditions. Under inert atmosphere, for example under Argon atmosphere, a compound of formula (IX) and a base, for example triethylamine or pyridine may be cooled to a temperature between -10 and 10°C, in particular 0°C, in a polar solvent, for example DCM, ethyl acetate, diethyl ether, tetrahydrofuran or acetone. A reagent, for example Ac₂O or acetyl chloride may be added dropwise. After addition, the mixture may be heated to reflux during several hours, preferably 2 hours, before cooling at room temperature. A saturate salt solution, for example of Na₂CO₃, NaHCO₃, KOH or NaOH may be added to neutralize the mixture before separating the phases.

A compound of formula (VII) can be converted in STEP 2 to a compound of formula (VI) under diazotisation and reduction conditions. To a solution of a compound of formula (VII) in a solution of acid, for example a solution of aqueous HCl, HBr or H₂SO₄ at cold temperature, as 0°C, may be slowly added a solution of nitrite source, for example sodium nitrite or ammonium nitrite in water. Then the mixture may be stirred during approximatively 30 min before slow addition of a solution of stannous chloride dihydrate in HCl or sodium sulfite in water. The mixture may be stirred, still at cool temperature for 2 hours and may be then basified to pH = 12 with, for example a solution of KOH or NaOH.

Compounds of formula (VIII) and (VI) may be reacted together in STEP 3 to give a compound of formula (V) under Fischer reaction conditions. To solution of a compound of formula (VIII) in aqueous acid solution, for example in a solution of aqueous H₂SO₄ , HCl or acetic acid may be added 1.0eq of a compound of formula (VI). This mixture may be heated to reflux for between 2 and 3 hours, preferably for 2.30 hours before cooling at room temperature. A saturate salt solution, for example of Na₂CO₃, NaHCO₃, KOH or NaOH may be added to neutralize the mixture before extracting and combining organic phases.

A compound of formula (V) may be converted in STEP 4 to a compound of formula (III) under deprotection conditions. A solution of a compound of formula (V) in aqueous acid solution, for example in a solution of aqueous H₂SO₄ or HCl, may be heated to reflux for few hours, preferably 16 hours and may be then cool to room temperature. A salt solution, for example of NaOH, Na₂CO₃, NaHCO₃ or KOH may be added to neutralize the mixture until reaching a pH of between 9 and 10 before extracting and combining organic phases.

Depending on the radicals of compounds of formula (IV), final reactions can be performed in one, two or three steps, using a microwave reactor or not.

A compound of formula (III) may be converted in STEP 5 to a compound of formula (II) by reacting a compound of formula (III) with a compound of formula (IV) under condensation conditions. Under inert gas, for example under Argon atmosphere, a compound of formula (III) may be put in solution in an organic solvent, preferably an aprotic polar solvent, for example DCM, tetrahydrofuran or diethyl ether with an aldehyde for example naphtaldehyde. To the mixture may be added a desiccant, for example anhydrous sodium sulfate, anhydrous magnesium sulfate, or molecular sieves and then the mixture may be stirred at room temperature for several hours, for example 16 hours, then filtrated and concentrated *in vacuo.*

A compound of formula (II) may be converted in STEP 6 to a compound of formula (Ia), wherein 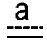 and 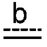 both represent a single bond and R₂ is a hydrogen atom, under Pictet-Spengler conditions. The Pictet-Spengler conditions may in particular be described in Molecules 2020, 25(2), 414 (https://doi.org/10.3390/molecules25020414) or in Current Pharmaceutical Design, 2016, 22, 1808-1850 (https://doi.org/10.2174/1381612822666151231100247).

To a solution of a compound of formula (II) and in a dry organic solvent, for example THF (tetrahydrofurane), DCM (dichloromethane) or toluene under inert atmosphere, for example under Argon and in a dry tube, may be added a catalyst, for example AlCl₃ or BF₃Et₂. The tube may be sealed and heated to a temperature between 40°C and 80°C, for example 68°C, for between 14 and 18 hours, for example 16 hours, or may be submitted to microwave irradiation to a temperature between 100°C and 200°C, for example 150°C, for between 45 and 90 minutes, for example 60 minutes, before cooling at room temperature. The mixture may be then treated with saturated salt solution, for example a saturated solution of NaHCO₃.

A compound of formula (Ia) may be converted in STEP 7 to a compound of formula (Ib), wherein 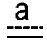 and 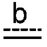 both represent a double bond and R₂ does not exist, under aromatization conditions. To a solution of a compound of formula (Ia) in an organic solvent, for example in acetone, may be added a heterogeneous oxidant, such as potassium permanganate or copper bromide in presence of air. The mixture may be stirred at room temperature for at least 24 hours, preferably at least 48 hours, then filtrated, on Celite for example. The filtrate may then be treated for example by extraction procedure under classical conditions.

### SCHEME 2

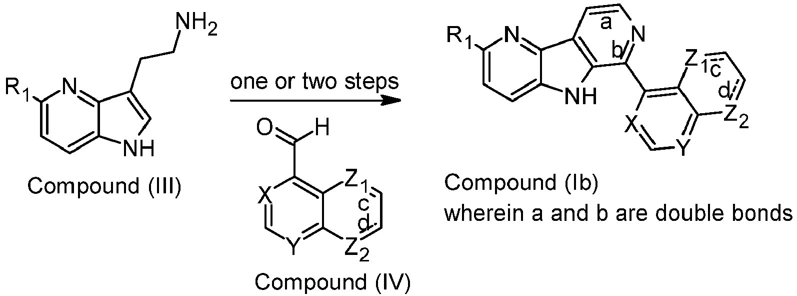

SCHEME 2 represents an alternative way for obtaining a compound of formula (Ib) as defined above.

A compound of formula (III) may be converted in two steps to a compound of formula (Ib). To a solution of a compound of formula (III) and a compound of formula (IV) as defined above, in a dry organic solvent, such as dry THF, DCM or toluene, placed under inert atmosphere, for example under Argon, may be added an acid, for example TFA (trifluoroacetic acid), p-TSOH (p-toluensolfonic acid) or DPP (diphenylphosphate). The reaction may be stirred at cool temperature during few hours, preferably at 0°C during 1 hour before warming up to room temperature. To the mixture may be added a saturated salt solution, for example a saturated solution of NaHCO₃ and organic phases are then extracted, dried, filtrated and concentrated *in vacuo.*

The residue may be secondly diluted in a solvent, for example in xylene, mesitylene, EtOH (ethanol) or octane. The mixture may be degassed before adding a catalyst as Pd/C. The mixture may be heated to reflux for more than 8 hours, for example 16 hours, then cool to room temperature and filtrated, on Celite for example.

According to an alternative route, a compound of formula (III) may be directly converted in one step to a compound of formula (Ib) by using a microwave reactor. A compound of formula (III) and of formula (IV) may be put in a dry microwave tube with an aprotic polar solvent, for example 1,2-dichloroethane, DCM or THF. To this mixture may be added a catalyst, for example a mixture of ytterbium triflate and trimethysilyl chloride, ytterbium triflate, aluminium chloride, titanium chloride or other Lewis acid or Pd/C in the presence of lithium carbonate. The tube may be then sealed and heated to between 100°C and 200°C, for example 150°C under microwave irradiation for between 30 minutes and 1 hour, for example 40 minutes and may be then cooled to room temperature. The mixture may be treated with saturated salt solution, for example a saturated solution of NaHCO₃ and organic phases are then extracted, dried, filtrated and concentrated *in vacuo.*

Herein is also provided a process for preparing a compound of formula (I) as defined above or anyone of its pharmaceutically acceptable salts, wherein it comprises at least a step of reacting a compound of formula (II) 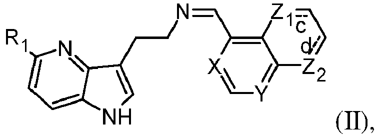 wherein R₁, X, Y, Z₁, Z₂, 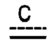 and 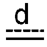 are as defined above,
under Pictet-Spengler conditions, in particular in an organic solvent, under inert atmosphere, and in presence of a catalyst, such as AlCl₃ or BF₃Et₂, in particular under a temperature between 40 and 68°C, for between 14 and 18 hours, before cooling a room temperature, for obtaining a compound of formula (Ia), wherein 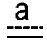 and 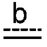 both represent single bonds and 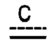 and 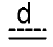 independently represent a single or double bond , which is optionally placed under aromatization conditions, in particular in an organic solvent, in presence of an heterogeneous oxidant such as potassium permanganate or copper bromide, in particular at room temperature for at least 24 hours, for obtaining a compound of formula (Ib), wherein 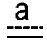 and 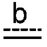 both represent double bonds and 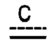 and 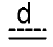 independently represent a single or double bond.

Herein is also provided a process for preparing a compound of formula compound (I), wherein 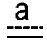 and 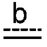 both represent a double bond and R₂ does not exist as defined above or anyone of its pharmaceutically acceptable salts, wherein it comprises at least a step of reacting a compound of formula (III) 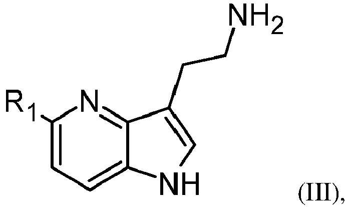 wherein R₁ is as defined above, with a compound of formula (IV) 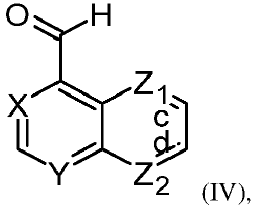 wherein, X, Y, Z₁, Z₂, 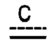 and 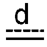 are as defined above,
successively in presence of an acid, such as trifluoroacetic acid, p-toluensolfonic acid or diphenylphosphate, under inert atmosphere, and in a second step in a solvent, such as xylene, mesitylene, EtOH or octane, in presence of a catalyst such as Pd/C, under heating to reflux, in particular for more than 8 hours, or alternatively under microwave, in an aprotic polar solvent, in presence of a catalyst, such as a mixture of ytterbium triflate and trimethysilyl chloride, ytterbium triflate, aluminium chloride, titanium chloride or other Lewis acid or Pd/C in the presence of lithium carbonate, under heat, in particular at a temperature between 100°C and 200°C.

The chemical structures and spectroscopic data of some compounds of formula (I) of the invention are illustrated respectively in the following Table I and Table II.

**Table I**

| No | Structure |
|---|---|
| 1 (ex 1) | 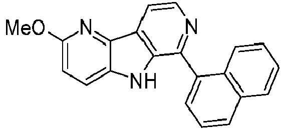 |
| 2 (ex 2) | 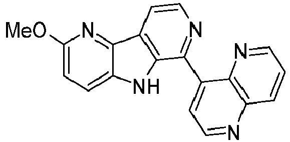 |
| 3 (ex 3) | 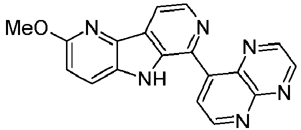 |
| 4 (ex 4) | 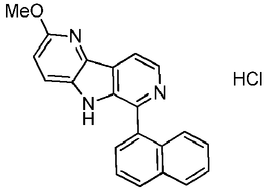 |
| 5 | 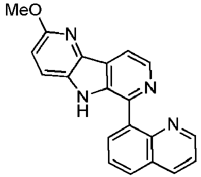 |
| 6 | 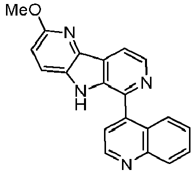 |
| 7 | 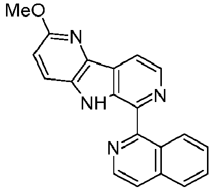 |
| 8 | 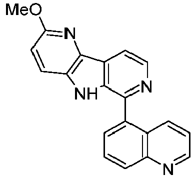 |
| 9 | 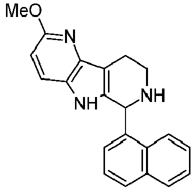 |
| 10 | 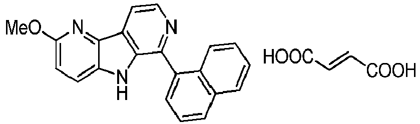 |
| 11 | 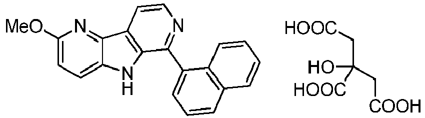 |
| 12 | 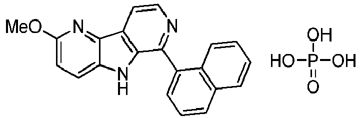 |
| 13 | 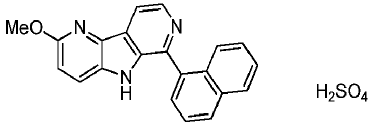 |
| 14 | 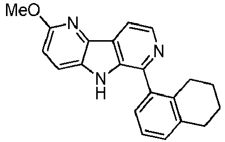 |
| 15 | 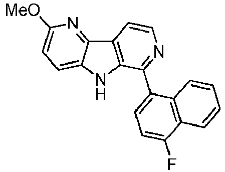 |
| 16 | 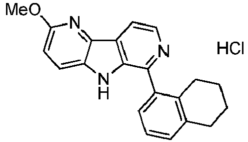 |
| 17 | 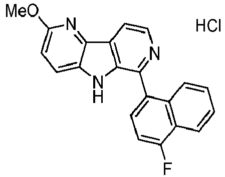 |

| | **Table II** |
|---|---|
| **Ex** | **Characterizations** |
| **1** | ¹H NMR (400 MHz, Chloroform-*d*) δ 8.67 (d, *J* = 5.3 Hz, 1H), 8.20 (d, *J* = 5.3 Hz, 1H), 8.02 (d, *J* = 8.2 Hz, 1H), 7.98 (d, *J* = 8.2 Hz, 1H), 7.89 (s, 1H), 7.78 (d, *J* = 6.9 Hz, 1H), 7.75 (d, *J* = 8.5 Hz, 1H), 7.65 (t, *J* = 7.6 Hz, 1H), 7.61 (d, *J* = 8.8 Hz, 1H), 7.54 (t, *J* = 7.5 Hz, 1H), 7.43 (t, *J* = 7.6 Hz, 1H), 6.94 (d, *J* = 8.9 Hz, 1H), 4.12 (s, 3H). |
| | [M+H]⁺ = 326.1285 |
| **2** | ¹H NMR (400 MHz, Chloroform-d) δ 10.30 (brs, 1H), 9.19 (d, J = 4.5 Hz, 1H), 9.09 (dd, J = 4.2, 1.8 Hz, 1H), 8.72 (d, J = 5.1 Hz, 1H), 8.64 (dd, J = 8.5, 1.8 Hz, 1H), 8.42 (d, J = 4.5 Hz, 1H), 8.29 (d, J = 5.1 Hz, 1H), 7.81 (dd, J = 8.6, 4.2 Hz, 1H), 7.77 (d, J = 8.8 Hz, 1H), 6.99 (d, J = 8.8 Hz, 1H), 4.13 (s, 3H). |
| | [M+H]⁺ =328.1194 |
| **3** | ¹H NMR (400 MHz, DMSO-d6) δ 11.04 (s, 1H), 9.38 (d, J = 4.3 Hz, 1H), 9.23 (d, J = 1.6 Hz, 1H), 8.97 (d, J = 1.6 Hz, 1H), 8.54 (d, J = 5.3 Hz, 1H), 8.18 (d, J = 5.3 Hz, 1H), 8.10 (d, J = 4.3 Hz, 1H), 7.80 (d, J = 8.9 Hz, 1H), 7.02 (d, J = 8.8 Hz, 1H), 4.02 (s, 3H). |
| | [M+H]⁺ =329.1142 |
| **4** | ¹H NMR (250 MHz, DMSO-d6) δ 12.49 (s, 1H), 8.67 (q, J = 6.2 Hz, 2H), 8.37 (d, J = 8.2 Hz, 1H), 8.21 (d, J = 8.2 Hz, 1H), 8.11 - 7.95 (m, 2H), 7.85 (dd, J = 8.2, 7.1 Hz, 1H), 7.74 - 7.62 (m, 1H), 7.62 - 7.45 (m, 2H), 7.28 (d, J = 9.1 Hz, 1H), 4.08 (s, 3H). |
| | [M+H]⁺ = 326.1289 |
| **5** | ¹H NMR (400 MHz, Chloroform-d) δ 9.42 (s, 1H), 9.02 - 8.91 (m, 1H), 8.71 - 8.61 (m, 1H), 8.42 - 8.29 (m, 2H), 8.23 - 8.15 (m, 1H), 7.99 (d, *J* = 8.1 Hz, 1H), 7.77 (t, *J* = 7.7 Hz, 1H), 7.68 (d, *J* = 8.5 Hz, 1H), 7.57 - 7.48 (m, 1H), 6.93 (d, *J* = 8.8 Hz, 1H), 4.12 (s, 2H). |
| | [M+H]⁺ =327.1243 |
| **6** | ¹H NMR (400 MHz, Chloroform-*d*) δ 11.20 (s, 1H), 8.66 (d, *J* = 5.3 Hz, 1H), 8.43 (d, *J* = 4.4 Hz, 1H), 8.31 (d, *J* = 5.4 Hz, 1H), 7.91 (d, *J* = 8.9 Hz, 1H), 7.82 -7.75 (m, 1H), 7.71 (dd, *J* = 8.4, 1.6 Hz, 1H), 7.52 - 7.42 (m, 2H), 7.42 - 7.36 (m, 1H), 7.03 (d, *J* = 8.8 Hz, 1H), 4.17 (s, 3H). |
| | [M+H]⁺ =327.1236 |
| **7** | ¹H NMR (400 MHz, Chloroform-*d*) δ 10.77 (s, 1H), 9.85 - 9.75 (m, 1H), 8.71 (dd, *J* = 6.5, 5.3 Hz, 2H), 8.24 (dd, *J* = 5.2, 0.7 Hz, 1H), 7.91 (dd, *J* = 7.1, 2.6 Hz, 1H), 7.84 (d, *J* = 8.8 Hz, 1H), 7.79 - 7.64 (m, 3H), 6.99 (d, *J* = 8.9 Hz, 1H), 4.13 (s, 3H). |
| | [M+H]⁺ =327.1244 |
| **8** | ¹H NMR (400 MHz, DMSO-*d*₆) δ 11.29 (s, 1H), 8.97 (dd, *J* = 4.2, 1.7 Hz, 1H), 8.54 (d, *J* = 5.4 Hz, 1H), 8.28 - 8.19 (m, 1H), 8.18 - 8.14 (m, 1H), 8.13 (d, *J* = 5.4 Hz, 1H), 7.98 (dd, *J* = 8.4, 7.1 Hz, 1H), 7.92 (dd, *J* = 7.1, 1.3 Hz, 1H), 7.86 (d, *J* = 8.8 Hz, 1H), 7.49 (dd, *J* = 8.6, 4.2 Hz, 1H), 7.04 (d, *J* = 8.9 Hz, 1H), 4.03 (s, 3H). |
| | [M+H]⁺ =327.1241 |
| **9** | ¹H NMR (400 MHz, Chloroform-*d*) δ 8.33 - 8.19 (m, 1H), 7.92 - 7.87 (m, 1H), 7.84 (d, *J* = 8.3 Hz, 1H), 7.50 (s, 2H), 7.45 - 7.29 (m, 4H), 6.52 (d, *J* = 8.6 Hz, 1H), 5.90 (s, 1H), 4.03 (s, 3H), 3.41 (d, *J* = 11.5 Hz, 1H), 3.21 (ddd, *J* = 12.7, 7.8, 5.3 Hz, 1H), 3.11 - 2.94 (m, 2H). |
| | [M+H]⁺ =330.1597 |
| **10** | ¹H NMR (400 MHz, DMSO-*d*₆) δ 13.15 (s, 2H), 11.18 (s, 1H), 8.52 (d, *J* = 5.3 Hz, 1H), 8.17-8.02 (m, 3H), 7.85 (d, *J* = 8.8 Hz, 1H), 7.80 - 7.64 (m, 3H), 7.62 - 7.51 (m, 1H), 7.49 - 7.37 (m, 1H), 7.02 (d, *J* = 8.8 Hz, 1H), 6.63 (s, 2H), 4.02 (s, 3H). |
| **11** | ¹H NMR (400 MHz, DMSO-*d*₆) δ 12.38 (s, 3H), 11.21 (s, 1H), 8.52 (d, *J* = 5.3 Hz, 1H), 8.18 - 8.04 (m, 3H), 7.85 (d, *J* = 8.9 Hz, 1H), 7.81 - 7.70 (m, 2H), 7.67 (d, *J =* 8.5 Hz, 1H), 7.58 (ddd, *J* = 8.1, 6.8, 1.2 Hz, 1H), 7.45 (ddd, *J* = 8.4, 6.8, 1.3 Hz, 1H), 7.03 (d, *J* = 8.8 Hz, 1H), 4.03 (s, 3H), 3.44 (q, *J* = 7.1 Hz, 1H), 2.75 (d, *J* = 15.6 Hz, 2H), 2.65 (d, *J* = 15.4 Hz, 2H). |
| **12** | ¹H NMR (400 MHz, DMSO-*d*₆) δ 11.18 (s, 1H), 8.52 (d, *J* = 5.3 Hz, 1H), 8.19 - 8.02 (m, 3H), 7.85 (d, *J* = 8.9 Hz, 1H), 7.79 - 7.70 (m, 2H), 7.67 (d, *J* = 8.4 Hz, 1H), 7.58 (ddd, *J* = 8.2, 6.8, 1.2 Hz, 1H), 7.45 (ddd, *J* = 8.3, 6.8, 1.3 Hz, 1H), 7.03 (d, *J* = 8.9 Hz, 1H), 4.03 (s, 3H). |
| **13** | ¹H NMR (400 MHz, DMSO-*d*₆) δ 12.49 (s, 1H), 8.73 - 8.62 (m, 2H), 8.37 (d, *J* = 8.2 Hz, 1H), 8.22 (d, *J* = 8.2 Hz, 1H), 8.02 (d, *J* = 9.0 Hz, 2H), 7.86 (dd, *J* = 8.3, 7.1 Hz, 1H), 7.70 (ddd, *J* = 8.2, 6.7, 1.3 Hz, 1H), 7.63 - 7.54 (m, 1H), 7.51 (d, *J* = 8.3 Hz, 1H), 7.29 (d, *J* = 8.9 Hz, 1H), 4.09 (s, 3H). |
| **14** | ¹H NMR (400 MHz, Chloroform-*d*) δ 8.47 (d, *J* = 5.4 Hz, 1H), 8.22 (s, 1H), 8.07 (d, *J* = 5.4 Hz, 1H), 7.62 (d, *J* = 8.8 Hz, 1H), 7.31 - 7.12 (m, 3H), 6.92 (d, *J* = 8.8 Hz, 1H), 4.10 (s, 3H), 2.86 (t, *J* = 6.4 Hz, 2H), 2.67 - 2.44 (m, 2H), 1.85 - 1.72 (m, 2H), 1.72 - 1.61 (m, 2H). |
| | [M+H]⁺ = 344.1195 |
| **15** | ¹H NMR (400 MHz, Chloroform-*d*) δ 8.57 - 8.43 (m, 2H), 8.19 - 8.08 (m, 2H), 7.69 (d, *J* = 8.7 Hz, 1H), 7.61 - 7.47 (m, 3H), 7.42 (t, *J* = 7.6 Hz, 1H), 7.15 (dd, *J* = 10.2, 7.8 Hz, 1H), 6.87 (d, *J* = 8.8 Hz, 1H), 4.10 (s, 3H). |
| | [M+H]⁺ = 330.16028 |
| **16** | ¹H NMR (400 MHz, DMSO-*d*₆) δ 12.53 (s, 1H), 8.64 - 8.49 (m, 2H), 8.07 (d, *J* = 9.0 Hz, 1H), 7.55 - 7.37 (m, 3H), 7.28 (d, *J* = 9.0 Hz, 1H), 4.06 (s, 3H), 2.91 (t, *J* = 5.9 Hz, 2H), 2.44 (s, 2H), 1.87 - 1.72 (m, 2H), 1.72 - 1.61 (m, 2H). |
| **17** | ¹H NMR (400 MHz, DMSO-*d*₆) δ 12.46 (s, 1H), 8.70 - 8.59 (m, 2H), 8.31 (d, *J* = 8.1 Hz, 1H), 8.03 (dd, *J* = 8.6, 6.2 Hz, 2H), 7.80 (t, *J* = 7.6 Hz, 1H), 7.75 - 7.62 (m, 2H), 7.57 (d, *J* = 8.3 Hz, 1H), 7.26 (d, *J* = 9.0 Hz, 1H), 4.08 (s, 3H). |

### PATHOLOGIES

All the compounds disclosed in the present application are specifically considered herein for the treatment and/or prevention of cancer, in particular cancer expressing chromatin-bound MDM2 or C-MDM2 or cancers exhibiting recruitment of MDM2 to chromatin.

They inhibit C-MDM2 expression and promote C-MDM2 autoubiquitination and proteasomal degradation as demonstrated in the examples. They may be used in the treatment and/or prevention of cancer expressing C-MDM2, said cancer being dependent or independent of p53 and regardless of the p53 status of the cancer cells. The present invention also relates to prophylactic treatment of a cancer disease, e.g., in an individual suspected to have the cancer disease, or at risk for developing the cancer disease, in particular cancer expressing chromatin-bound MDM2 or C-MDM2 or cancer exhibiting recruitment of MDM2 to chromatin.

Without wishing to be bound by any particular theory, it is specified that a cancer exhibiting recruitment of MDM2 to chromatin does not equate to cancer exhibiting a MDM2 overexpression in the cell. A cancer exhibiting recruitment of MDM2 to chromatin encompass both (i) cancers exhibiting overexpression of MDM2 and (ii) cancers that do not exhibit MDM2 overexpression. A cancer exhibiting recruitment of MDM2 to chromatin may be a cancer wherein the cancer cells exhibit a MDM2 overexpression in the cytoplasm and wherein at least a part of the cellular MDM2 is localized in the cell nucleus. A cancer exhibiting recruitment of MDM2 to chromatin may also be a cancer wherein MDM2 is not overexpressed in the cancer cells and wherein at least part of the cellular MDM2 is localized in the cell nucleus.

Accordingly, in some embodiments, a cancer exhibiting recruitment of MDM2 to chromatin may not be a cancer exhibiting a MDM2 overexpression in the cytoplasm.

A cancer exhibiting recruitment of MDM2 to chromatin may be diagnosed with, for instance, the method of diagnosis disclosed in WO2019/106126 or in Cissé et al. (2020, Sci Trans Med, Vol. 12 ; 547).

In some embodiments, a cancer exhibiting recruitment of MDM2 to chromatin may be diagnosed in a subject with any method allowing to observe the localization of a protein, in particular MDM2, in a cancer cell sample or a cancer tissue sample.

Determination of a cancer exhibiting recruitment of MDM2 to chromatin in a subject may be also a mean of classifying a subject according to the type of cancer he is affected by. In particular, a subject may be classified as being affected with a cancer exhibiting a recruitment of MDM2 to chromatin or as being affected by a cancer that do not exhibit a recruitment of MDM2 to chromatin.

For instance, methods allowing to determine the localization of a protein, in particular MDM2, in a cancer cell sample or a cancer tissue sample may be made by immunofluorescence, in particular by microscopy, or by immunohistochemistry.

In some particular embodiments, a method for determining cancers exhibiting recruitment of MDM2 to chromatin in a subject in need thereof, may comprise the steps of: a) providing a cancer cell sample obtained from said subject, b) determining, in particular by immunofluorescence or immunohistochemistry, the localization of MDM2 in cancer cells, and c) concluding that the subject have a cancer exhibiting recruitment of MDM2 to chromatin when MDM2 is localized at nucleus (and hence on chromatin) or concluding that the subject do not have a cancer exhibiting recruitment of MDM2 to chromatin when MDM2 is not localized in the nucleus, in particular when MDM2 is localized only in the cytoplasm of the cancer cell.

Illustratively, the localization of MDM2 in cancer cells of a subject may be determined by immunohistochemistry. Cancer cells or tissues are collected from the tumor in the subject. The cancer cells are placed on a solid support. Then, an anti-MDM2 antibody is added to the cancer cells preparation. Subsequently, it is added a secondary antibody bound to a detection system (enzyme bound to the antibody, whose presence in the substrate causes a colored (peroxidase) or fluorescent (Rhodamine) reaction), causing a signal visible to the naked eye, or by microscopy and spectrophotometry techniques. Subsequently, it may be determined, through the observation of the signal color, whether MDM2 is localized at the nucleus (and hence on chromatin), or in the cytoplasm.

In some other embodiments, a method for diagnosing cancers exhibiting recruitment of MDM2 to chromatin in a subject in need thereof, may comprise the steps of: i) determining the level of nuclear-bound MDM2 in the biological sample, in particular the level of chromatin-bound MDM2, ii) concluding that the subject is affected by a cancer exhibiting recruitment of MDM2 to chromatin when the proportion of nuclear-bound MDM2 cells determined at step ii) represents more than about 1% of the cancer cells in the sample.

In some particular embodiments, a method for diagnosing cancers exhibiting recruitment of MDM2 to chromatin in a subject in need thereof, may comprise the steps of:
i) determining the level of nuclear-bound MDM2 in the cancer cells of the biological sample,
ii) concluding that the subject is affected by a cancer exhibiting recruitment of MDM2 to chromatin when the proportion of nuclear-bound MDM2 cells determined at step ii) represents more than about 1% of the cancer cells in the sample.

Assessing the level of nuclear-bound MDM2, in particular chromatin-bound MDM2, in cancer cells of a biological sample of a subject can be readily determined by a skilled person in the art.

The level of nuclear-bound MDM2, in a biological sample of a subject may be also determined in patient-derived tumor samples by immunoblot on cellular fractionation isolating chromatin.

In some embodiments, a biological sample may be a tissue sample.

In particular, a tissue sample may be a cancer tissue sample of the subject. Techniques for the collection of a tissue sample of a subject are well-known by a skilled person in the art. For instance, the collection of a tissue sample may be realized by a biopsy.

The methods of diagnosis described herein may be implemented as a biomarker test to determine if a subject is suffering from a cancer exhibiting recruitment of MDM2 to chromatin.

In some embodiments, the methods of diagnosis described herein provide clinical information. Illustratively, the methods of diagnosis as described herein allow completing information relating to a biopsy sample provided from a subject suffering from a cancer exhibiting recruitment of MDM2 to chromatin.

Illustratively, the methods of diagnosis described herein allow determining the presence of MDM2 to chromatin in a subject suffering from a cancer, wherein the detection of MDM2 to chromatin may permit the medical practitioner to decide to administer a C-MDM2 expression inhibitor of the present disclosure to the subject suffering from cancer.

According to the present disclosure, a recruitment of MDM2 to chromatin in the cancer cells of a subject in need thereof, may occur only after a certain period subsequent to the occurrence of the cancer or in contrast may occur immediately upon the occurrence of the cancer. In particular, MDM2 recruitment to chromatin may occur at the onset of the cancer but also during the different phases of the cancer, including following administration to the said subject of at least a first treatment against cancer.

In some embodiments, a cancer expressing C-MDM2 as disclosed herein may be selected from the group consisting of bone cancer, brain cancer, ovary cancer, breast cancer, lung cancer, colorectal cancer, osteosarcoma, skin cancer, blood cancer, including acute myeloid leukemia, pancreatic cancer, prostate cancer and liposarcoma, more particularly selected from the group consisting of skin cancer, liposarcoma and blood cancer, for example but not limited to liposarcoma, melanoma and acute myeloid leukemia. In some embodiments, a cancer expressing C-MDM2 as disclosed herein may be liposarcoma.

The term "liposarcoma" or "LPS" has its general meaning in the art and refers to soft tissue sarcomas of mesenchymal origin such as revised in the World Health Organisation Classification ICD10 C49.9. The term "liposarcoma" also refers to well-differentiated and de-differentiated liposarcoma (WD- and DD-LPS). The term "liposarcoma" also relates to Malignant mesenchymal neoplasms, a type of soft tissue sarcoma, a group consisting of lipomatous tumors of varying severity ranging from slowgrowing to aggressive and metastatic. Liposarcomas are most often located in the lower extremities or retroperitoneum, but they can also occur in the upper extremities, neck, peritoneal cavity, spermatic cord, breast, vulva and axilla. The term "liposarcoma" also relates to dedifferentiated liposarcoma and well-differentiated liposarcoma.

In some embodiments, the term "liposarcoma" refers to liposarcoma exhibiting recruitment of MDM2 to chromatin.

The compounds according to the present invention may also be useful for decreasing tumor growth and inhibit metastasis.

The inventors have in addition demonstrated that the compounds of the invention may be particularly useful as a first-line treatment for melanoma, liposarcoma and acute myeloid leukemia.

They have further showed that patients who developed tumors and who have been treated in first-line by radiotherapy, chemotherapy and/or immunotherapies could advantageously be treated in a second line treatment by the compounds of the invention. They indeed showed that said tumor cells were thereafter sensitive to the C-MDM2 expression inhibitors, and more particularly to the compounds of the present invention.

In the framework of the present invention, first-line treatment of cancer refers to the initial, or first treatment recommended for said cancer. This may also be referred to as primary treatment, initial treatment, or induction therapy. First-line treatment for cancer is the one that, for most people, is expected to provide the best results with the fewest number of side effects. In contrast, second-line cancer treatments are used when the first-line treatment failed to improve said cancer, or if it worked for a while and then the cancer progressed, and tend to be less effective.

Thus, in one embodiment, herein is provided a compound of formula (I) according to the present invention for use in the treatment of a cancer expressing chromatin-bound MDM2 or C-MDM2 or a cancer exhibiting recruitment of MDM2 to chromatin, in particular selected from melanoma, liposarcoma and acute myeloid leukemia, in a patient who has not previously been treated by another anti-cancer drug.

In another embodiment, herein is provided a compound of formula (I) according to the present invention for use in the treatment of a tumor, including tumor not spontaneously expressing chromatin-bound MDM2 or C-MDM2 or a tumor not exhibiting recruitment of MDM2 to chromatin before any treatment but exhibiting recruitment of MDM2 to chromatin after treatment, in a patient who has been previously treated by radiotherapy, chemotherapy and/or immunotherapies.

### Pharmaceutical compositions

In a further aspect, the present disclosure relates to the administration of a compound of formula (I) as described herein in the form of a pharmaceutical composition. Otherwise said, the present disclosure also relates to pharmaceutical compositions comprising a compound of formula (I) as described herein.

Typically, a compound of formula (I) as described herein may be combined with pharmaceutically or physiologically acceptable excipients or carrier, and optionally sustained-release matrices, such as biodegradable polymers, to form therapeutic compositions.

Pharmaceutical compositions provided herein comprise the active agents, i.e., a compound of formula (I) as described herein, in a therapeutically effective amount, i.e., in an amount effective to achieve its intended purpose. As exposed above, the actual effective amount for a particular application will depend, inter alia, on the condition being treated and various other factors well-known in the art such as the age, the weight, the sex of the patient, the presence of other potential aggravating conditions, or the diet. Determination of a therapeutically effective amount of a compound of formula (I) as described herein is well within the capabilities of those skilled in the art.

The pharmaceutical compositions described herein can be prepared according to techniques known to the skilled person by using a compound of formula (I) as described herein in association with a pharmaceutically acceptable excipient or carrier.

These pharmaceutical compositions may comprise one or more pharmaceutically acceptable excipients or carriers. Suitable carriers and excipients and their formulations are described, for example, in Remington: The Science and Practice of Pharmacy, 21st Edition, David B. Troy, ed., Lippicott Williams & Wilkins (2005). By pharmaceutically acceptable carrier is meant a material that is not biologically or otherwise undesirable, i.e., the material is administered to a subject without causing undesirable biological effects or interacting in a deleterious manner with the other components of the pharmaceutical composition in which it is contained.

The pharmaceutical compositions can be in any form deemed appropriate by the skilled person, such as solid, semi-solid, liquid, granular, inhalation or aerosol inhalation.

The liquid forms may be appropriate forms for oral or systemic administration.

Pharmaceutical compositions suitable for oral administration can be capsules, tablets, pills, powders, granules, solutions or suspensions in aqueous or non-aqueous liquids, foam or beaten edible, liquid oil in water emulsions or liquid water in oil emulsions.

For example, for oral administration in capsule or tablet form, the active agents mentioned herein may be combined with pharmaceutically acceptable inert carrier such as ethanol, glycerol, water and similar. There may also be present flavourings, preservative, coloring coating and/or dispersant agents.

Pharmaceutical compositions suitable for parenteral administration may include sterile aqueous or non-aqueous solution for injection which may contain antioxidants, buffers, bacteriostatic and solutes which render the solution isotonic with the blood of the intended recipient, and aqueous or non-aqueous sterile suspensions which may include suspending and thickening agents. These compositions may be sterilized by conventional, well known sterilization techniques.

A parenteral composition may include a solution or suspension of the compounds in a vehicle such as sterile water or a parenterally acceptable oil. Alternatively, the solution can be lyophilized. The lyophilized parenteral pharmaceutical composition can be reconstituted with a suitable solvent just prior to administration.

The pharmaceutical compositions may be presented in single dose or multi-dose containers, for example, sealed ampoules or vials, and may be stored in lyophilized condition requiring only the addition of the sterile liquid carrier, for example water for injections, immediately prior to use. Extemporaneous injection solutions and suspensions may be prepared from powders, granules, lyophilized and sterile compresses.

In the case of parenteral administration, the composition may also be provided with the active ingredients in separate containers that can be suitably admixed according to the desired dosage taking into account the weight, age, gender and health status of the patient in need thereof.

In all cases, the pharmaceutical compositions must be stable under the conditions of manufacture and storage and must be preserved against the contaminating action of microorganisms, such as bacteria and fungi.

All or part of the particular features and embodiments relating to pharmaceutical compositions according to the present disclosure, also apply to the intended uses and methods of the present disclosure.

In some embodiments, the present disclosure relates to the use of a compound of formula (I) according to the present disclosure for the manufacture of a medicament for the prevention and/or treatment of a cancer as described above, in particular liposarcoma, in a subject in need thereof, wherein the subject has been previously classified as being affected with a cancer exhibiting recruitment of MDM2 to chromatin.

The examples provided herein are intended to be merely exemplary, and those skilled in the art will recognize, or will be able to ascertain using no more than routine experimentation, numerous equivalents of specific compounds, materials, and procedures. All such equivalents are considered to be within the scope of the invention and are encompassed by the appended claims.

### EXAMPLES

In the examples, the following terms mean:
- M.S: Molecular sieves,
- DCM: Dichloromethane,
- THF: tetrahydrofurane,
- TFA: trifluoroacetic acid,
- RT: room temperature,
- DMEM: Dulbecco's Modified Eagle Medium.

### Preparation of intermediates:

### Synthesis of 2-(5-methoxy-1H-pyrrolo[3,2-b]pyridin-3-yl)ethanamine

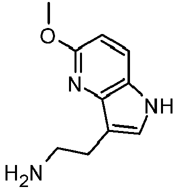

### Step 1: N-(4,4-diethoxybutyl)acetamide

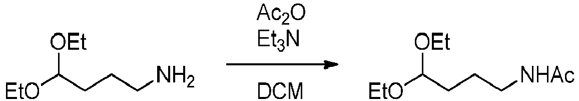

Under an Argon atmosphere, 3.6 mL of 4,4-diethoxybutan-1-amine (20.8 mmol) and 7.2 mL triethylamine (2.5 equiv.) are dissolved in 25 mL of dry DCM. The mixture is cooled to 0°C and 11.8 mL of Ac₂O (6 equiv.) are added dropwise. Once the addition is complete, the reaction is heated to reflux for 2 hours and then allowed to cool to room temperature. It is neutralized with saturated Na₂CO₃ solution and the phases are separated. The aqueous layer is extracted 3 times with DCM. The organic phases are gathered, washed with saturated Na₂CO₃ solution and brine, dried over anhydrous magnesium sulphate, filtrated and concentrated in vacuo to obtain N-(4,4-diethoxybutyl)acetamide which is used without further purification.

¹H NMR (250 MHz, Chloroform-d) δ 5.67 (s, 1H), 4.48 (t, J = 5.3 Hz, 1H), 3.65 (dq, J = 9.5, 7.1 Hz, 2H), 3.49 (dq, J = 9.4, 7.1 Hz, 2H), 3.35 - 3.18 (m, 2H), 1.96 (s, 3H), 1.73 - 1.50 (m, 4H), 1.20 (t, J = 7.1 Hz, 6H).

### Step 2: 5-hydrazinyl-2-methoxypyridine

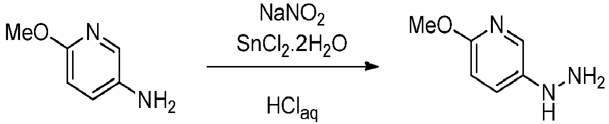

To a solution of 2.8 g 6-methoxypyridin-3-amine (22.5 mmol) in 28 mL of 6N aqueous HCl, is added at 0°C and dropwise a solution of 1.6 g of sodium nitrite (1 equiv.) in 14 mL of H₂O. The reaction is stirred at 0°C for 30 minutes then a solution of 12.7 g of tin chloride dihydrate in 28 mL of 6N aqueous HCl is slowly added at 0°C. The reaction mixture is stirred 2 more hours at 0°C. It is then basified with 40% KOH solution until pH=12 and extracted 3 times with EtOAc. The combined organic layers are dried over anhydrous magnesium sulphate, filtrated, and concentrated *in vacuo* to obtain 5-hydrazinyl-2-methoxypyridine which is used without further purification.

¹H NMR (250 MHz, Chloroform-d) δ 7.76 (d, J = 3.0 Hz, 1H), 7.18 (dd, J = 8.9, 3.0 Hz, 1H), 6.64 (dd, J = 8.8, 0.6 Hz, 1H), 4.96 (brs, 1H), 3.86 (s, 3H), 3.60(brs, 2H).

### Step 3: N-(2-(5-methoxy-1H-pyrrolo[3,2-b]pyridin-3-yl)ethyl)acetamide

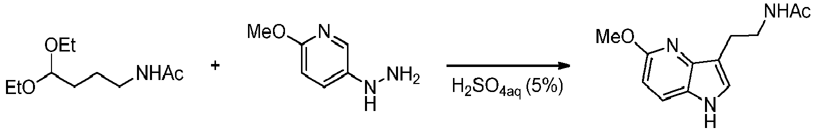

To a solution of 2.8 g of N-(4,4-diethoxybutyl)acetamide (13.9 mmol) in 40 mL of 5% aqueous H₂SO₄, is added 1.5 g of 5-hydrazinyl-2-methoxypyridine (1.0 equiv.). The reaction mixture is heated to reflux for 2h30 and then allowed to cool to room temperature. It is neutralized with saturated Na₂CO₃ solution and extracted 4 times with EtOAc. The combined organic layers are washed with H₂O, dried over anhydrous magnesium sulphate, filtrated and concentrated *in vacuo.*

The crude is purified by silica gel flash chromatography (100% EtOAc to 90-10 EtOAc/MeOH) to afford pureN-(2-(5-methoxy-1H-pyrrolo[3,2-b]pyridin-3-yl)ethyl)acetamide.

¹H NMR (250 MHz, Chloroform-d) δ 8.23 (s, 1H), 7.59 (d, J = 8.8 Hz, 1H), 7.40 - 7.28 (m, 1H), 7.16 (d, J = 2.7 Hz, 1H), 6.74 - 6.52 (m, 1H), 4.03 (s, 3H), 3.67 - 3.49 (m, 2H), 3.07 - 2.90 (m, 2H), 1.94 (s, 3H).

### Step 4: 2-(5-methoxy-1H-pyrrolo[3,2-b]pyridn-3-yl)ethanamine

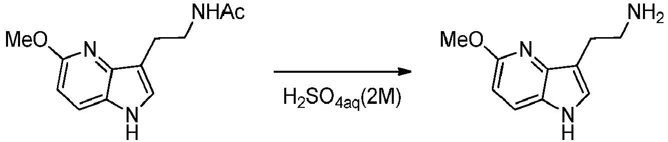

1.4 g of N-(2-(5-methoxy-1H-pyrrolo[3,2-b]pyridin-3-yl)ethyl)acetamide (6.1 mmol) are dissolved in 80 mL of 2M aqueous H₂SO₄. The reaction mixture is heated to reflux for 16 hours and then allowed to cool to room temperature. It is neutralized with 1M NaOH until pH = 9-10 and extracted 4 times with EtOAc. The combined organic layers are washed with H₂O, dried over anhydrous magnesium sulphate, filtrated and concentrated *in vacuo* to obtain pure 2-(5-methoxy-1H-pyrrolo[3,2-b]pyridin-3-yl)ethanamine.

¹H NMR (250 MHz, Chloroform-d) δ 8.32 (s, 1H), 7.52 (dd, J = 8.8, 0.6 Hz, 1H), 7.12 (s, 1H), 6.65 - 6.49 (m, 1H), 3.98 (d, J = 0.4 Hz, 3H), 3.14 - 3.04 (m, 2H), 2.93 (t, J = 6.3 Hz, 2H).

### EXAMPLE 1: 2-methoxy-6-(naphthalen-1-yl)-5H-pyrrolo[3,2-b:5,4-c'] dipyridine

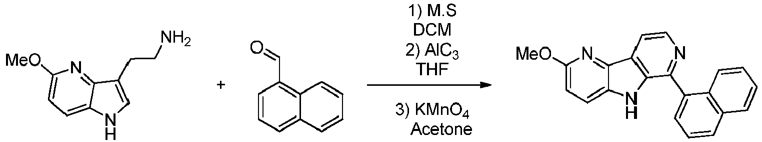

Step 1: Under an Argon atmosphere, to a solution of 363 mg of 2-(5-methoxy-1H-pyrrolo[3,2-b]pyridin-3-yl)ethanamine (1.9 mmol) and 284 µL of naphtaldehyde (1.1 equiv.) in 12 mL of dry DCM, is added molecular sieves. The reaction mixture is stirred at room temperature for 16 hours, then filtrated and concentrated *in vacuo* to afford crude imine which is used without further purification.

¹H NMR (250 MHz, Chloroform-d) δ 8.80 (s, 1H), 8.57 - 8.44 (m, 1H), 8.30 (s, 1H), 7.91 - 7.80 (m, 3H), 7.55 - 7.37 (m, 4H), 7.05 (d, J = 2.5 Hz, 1H), 6.59 (d, J = 8.8 Hz, 1H), 4.25 - 4.13 (m, 2H), 4.04 (s, 3H), 3.30 (t, J = 6.9 Hz, 2H).

Step 2: In a dry tube, to a solution of 600 mg of crude imine (1.9 mmol) in 20 mL of dry THF, is added under Argon 127 mg of aluminium trichloride (0.5 equiv.). The tube is sealed, heated to 68°C for 16 hours and then allowed to cool to room temperature. The reaction mixture is treated with saturated NaHCO₃ solution and extracted 3 times with EtOAc. The combined organic layers are dried over anhydrous magnesium sulphate, filtrated and concentrated *in vacuo* to obtain crude 2-methoxy-6-(naphthalen-1-yl)-6,7,8,9-tetrahydro-5H-pyrrolo[3,2-b:5,4-c']dipyridine which is used without further purification but can be purified by silica gel flash chromatography (99/1 DCM/MeOH to 90/10 DCM/MeOH).

¹H NMR (250 MHz, Chloroform-d) δ 8.31 - 8.14 (m, 1H), 8.02 - 7.75 (m, 2H), 7.61 - 7.44 (m, 3H), 7.44 - 7.28 (m, 3H), 6.51 (d, J = 8.7 Hz, 1H), 5.86 (brs, 1H), 4.02 (s, 3H), 3.49 - 3.31 (m, 1H), 3.26 - 3.09 (m, 1H), 3.09 - 2.94 (m, 2H).

Step 3: To a solution of 600 mg of crude 2-methoxy-6-(naphthalen-1-yl)-6,7,8,9-tetrahydro-5H-pyrrolo[3,2-b:5,4-c']dipyridine (1.8 mmol) in 87 mL of acetone, is added under air 2.9 g of potassium permanganate (10.0 equiv.). The reaction is stirred at room temperature for 48 hours and then filtrated on Celite. The filtrate is treated with saturated Na₂S₂O₃ solution and basified with 28% aqueous ammonia. The resulting solution is extracted 3 times with EtOAc. The combined organic layers are dried over anhydrous magnesium sulphate, filtrated and concentrated *in vacuo.* The crude is purified by silica gel flash chromatography (70/30 EtOAc /Petroleum Ether to 80/20 EtOAc/ Petroleum Ether) to afford pure 2-methoxy-6-(naphthalen-1-yl)-5H-pyrrolo[3,2-b:5,4-c']dipyridine.

¹H NMR (250 MHz, Chloroform-d) δ 8.65 (d, J = 5.3 Hz, 1H), 8.19 (dd, J = 5.3, 0.8 Hz, 1H), 8.04 - 7.94 (m, 2H), 7.93 (s, 1H), 7.81 - 7.70 (m, 2H), 7.68 - 7.57 (m, 2H), 7.57 - 7.50 (m, 1H), 7.42 (ddd, J = 8.5, 6.9, 1.4 Hz, 1H), 6.93 (d, J = 8.9 Hz, 1H), 4.12 (s, 3H).

### EXAMPLE 2: 2-methoxy-6-(1,5-naphthyridin-4-yl)-5H-pyrrolo[3,2-b:5,4-c'] dipyridine

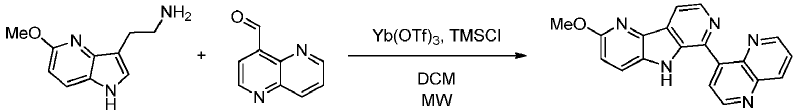

In a dry microwave tube, to a solution of 150 mg of 2-(5-methoxy-1H-pyrrolo[3,2-b]pyridin-3-yl)ethanamine (0.8 mmol) in dry 3.6 mL of dry DCM is added under Argon 124 mg of 1,5-naphthyridine-4-carbaldehyde (1.0 equiv.), 194 mg of ytterbium triflate (0.4 equiv.) and 0.1 mL of trimethylsilyl chloride (1.0 equiv.). The tube is sealed and heated to 150 °C under microwave irradiation for 40 minutes. Once cooled to room temperature, the reaction mixture is treated with saturated NaHCO₃ solution and extracted 4 times with DCM. The combined organic layers are dried over anhydrous magnesium sulphate, filtrated and concentrated *in vacuo.*

The crude is purified by silica gel chromatography (95/5 DCM/MeOH) to afford pure 2-methoxy-6-(1,5-naphthyridin-4-yl)-5H-pyrrolo[3,2-b:5,4-c']dipyridine.

¹H NMR (400 MHz, Chloroform-d) δ 10.30 (brs, 1H), 9.19 (d, J = 4.5 Hz, 1H), 9.09 (dd, J = 4.2, 1.8 Hz, 1H), 8.72 (d, J = 5.1 Hz, 1H), 8.64 (dd, J = 8.5, 1.8 Hz, 1H), 8.42 (d, J = 4.5 Hz, 1H), 8.29 (d, J = 5.1 Hz, 1H), 7.81 (dd, J = 8.6, 4.2 Hz, 1H), 7.77 (d, J = 8.8 Hz, 1H), 6.99 (d, J = 8.8 Hz, 1H), 4.13 (s, 3H).

### EXAMPLE 3: 2-methoxy-6-(pyrido[2,3-b]pyrazin-8-yl)-5H-pyrrolo[3,2-b:5,4-c'] dipyridine

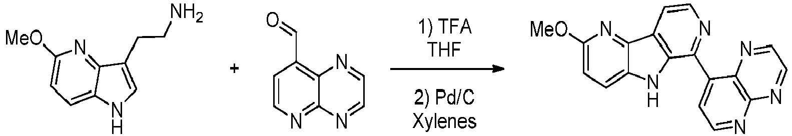

To a solution of 90 mg of 2-(5-methoxy-1H-pyrrolo[3,2-b]pyridin-3-yl)ethanamine (0.5 mmol) and 90 mg of pyrido[2,3-b]pyrazine-8-carbaldehyde (0.6 mmol) in 10 mL of dry THF is added under Argon at 0°C 0.2 mL of TFA. The reaction is stirred at 0°C for 1 hour and allowed to warm up to room temperature. Then saturated NaHCO₃ solution is added and the reaction is extracted with EtOAc three times. The combined organic layers are dried over anhydrous magnesium sulphate, filtrated and concentrated *in vacuo.*

The residue is diluted in 10 mL of xylene, the solution is degassed and 100 mg of 10% Pd/C is added. The reaction mixture is heated to reflux for 16 hours, then allowed to cool to room temperature and filtrated on celite. The filtrate is concentrated *in vacuo* and purified by silica gel chromatography (DCM/MeOH 90/10) to afford pure 2-methoxy-6-(pyrido[2,3-b]pyrazin-8-yl)-5H-pyrrolo[3,2-b:5,4-c']dipyridine.

¹H NMR (400 MHz, DMSO-d6) δ 11.04 (s, 1H), 9.38 (d, J = 4.3 Hz, 1H), 9.23 (d, J = 1.6 Hz, 1H), 8.97 (d, J = 1.6 Hz, 1H), 8.54 (d, J = 5.3 Hz, 1H), 8.18 (d, J = 5.3 Hz, 1H), 8.10 (d, J = 4.3 Hz, 1H), 7.80 (d, J = 8.9 Hz, 1H), 7.02 (d, J = 8.8 Hz, 1H), 4.02 (s, 3H).

### EXAMPLE 4: 2-methoxy-6-(naphthalen-1-yl)-5H-pyrrolo[3,2-b:5,4-c']dipyridine hydrochloride.

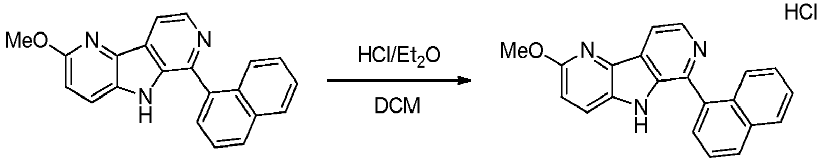

To a solution of 180 mg of 2-methoxy-6-(naphthalen-1-yl)-5H-pyrrolo[3,2-b:5,4-c']dipyridine (0.6 mmol) in dry DCM is added 0.6 mL of 1M HCl in Et₂O (1.1 equiv.). The reaction mixture is stirred at room temperature for 30 minutes and concentrated to dryness to afford pure 2-methoxy-6-(naphthalen-1-yl)-5H-pyrrolo[3,2-b:5,4-c']dipyridine hydrochloride.

¹H NMR (250 MHz, DMSO-d6) δ 12.49 (s, 1H), 8.67 (q, J = 6.2 Hz, 2H), 8.37 (d, J = 8.2 Hz, 1H), 8.21 (d, J = 8.2 Hz, 1H), 8.11 - 7.95 (m, 2H), 7.85 (dd, J = 8.2, 7.1 Hz, 1H), 7.74 - 7.62 (m, 1H), 7.62 - 7.45 (m, 2H), 7.28 (d, J = 9.1 Hz, 1H), 4.08 (s, 3H).

### Pharmacological data

The compounds of the invention have been the subject of pharmacological tests which have demonstrated their relevance as active substances in therapy and in particular for treating and/or preventing C-MDM2 cancers.

### EXAMPLE 5: In vitro anti-tumor effect on cell line dependent (IB111) or not (Hpac/ZR75.1) of C-MDM2

### A. Material and methods

Cancer cells exhibiting or not a recruitment of MDM2 to chromatin (C-MDM2) were treated for 72 hours with increasing concentrations of compounds of formula (I) according to the invention.

MDM2 degradation is measured by western blot and immunofluorescence.

Cells were seeded in 6-well plates (Sarsted) in complete DMEM medium as to obtain triplicates for each condition (500 000 cells/well). After 24 hours, the compounds were added to the cells. Then, 18 hours later cells were collected for western blotting or immunofluorescence.
- *Western Blot*: Cells were collected in Laemmli buffer, boiled and analyzed in 8% acrylamide gel.
- *Immunofluorescence*: Cancer cells were fixed with paraformaldehyde (PFA) 4% for 15 minutes then permeabilized for 15 minutes at RT with phosphate-buffered saline (PBS) Triton 0.1% and blocked with Phosphate buffered saline-Anti-Bovine Albumin (PBS-BSA) 0.3% for 1 hour at RT before overnight incubation at 4 °C with an anti- mouse monoclonal antibody against MDM2 (MABE340 Millipore). Immunodetection was performed using an Alexa 488-conjugated anti-mouse IgG antibody (Thermo Fisher) for 45 minutes at RT. Cover glasses were mounted with Mowiol (Biovalley) and DAPI (Sigma) before analysis on a Zeiss apotome.

### B. Results

Using the protocol described above, degradation was assessed, and results gathered in table III here below:

**Table III**

| | *Degradation efficacy is represented by:* | | | |
|---|---|---|---|---|
| | | | +++ ≥ *80*%, | |
| | | | *40*%≤++*<80*%, | |
| | | | *25*%≤+*<40*% | |
| | | | *<25%: no degradation: indicated* "-" *in the table* | |
| **compound No** | | **Chromatin-MDM2 degradation** | | **Cytoplasmic- MDM2 degradation** |
| **1** | | +++ | | - |
| **3** | | + | | - |
| **6** | | + | | - |
| **4** | | +++ | | - |
| **10** | | +++ | | - |
| **11** | | +++ | | - |
| **12** | | ++ | | - |
| **13** | | ++ | | - |
| **14** | | +++ | | - |
| **15** | | ++ | | - |
| **16** | | +++ | | - |
| **17** | | + | | - |

### C. Conclusion

Thus, experimental show that the compounds of formula (I) according to the present invention are C-MDM2 expression inhibitors.

### EXAMPLE 6: Cell survival after MDM2 inhibitor (IC₅₀)

### A. Material and methods

Cell survival after MDM2 inhibitor treatment (IC₅₀) was determined using the Sulforhodamine B assay (SRB assay).

Cancer cells exhibiting or not a recruitment of MDM2 to chromatin were treated for 72 hours with increasing concentrations of compounds.

Cells were seeded in 96-well plates (Sarsted) in complete DMEM medium as to obtain triplicates for each condition (5 000 cells/well). After 24 hours, serial dilutions of the indicated compounds were added to the cells. Then 48 hours later, cells were fixed by adding a 10% Trichloroacetic acid solution and stained with a 0,4% SRB solution in 1% acetic acid. Fixed SRB was finally dissolved in 10 mM Tris-HCl solution and 560nm absorbance was read using a PHERAstar FSX plate reader.

### B. Results

It was observed that compounds (1), (4), (10), (11), (12), (13), (14), (15) and (16) have an IC₅₀ <500nM on cancer cells exhibiting recruitment of MDM2 to chromatin (IB111). Moreover, said compounds were not efficient on cancer cells not exhibiting recruitment of MDM2 to chromatin (Hpac/ZR75.1).

### C. Conclusion

Thus, experimental evidence (i) shows that the compounds according to the invention may be useful for treating and/or preventing cancer and (ii) their selectivity with respect to cancer exhibiting recruitment of MDM2 to chromatin, and more particularly liposarcoma.

### EXAMPLE 7: In vivo evaluation of the anti-tumor efficacy

In order to support the *in vitro* results, *in vivo* evaluation of the anti-tumor efficacy was performed on one of the compounds of formula (I) according to the present invention having evidenced the antitumoral activity in examples 5 and 6 above.

### A. Material and methods

10 mice per group were engrafted with liposarcoma cells IB111. Mice were housed in a pathogen-free barrier facility in accordance with the regional ethics committee for animal warfare (n°CEEA-LR-12067). When tumor reached 100 mm³, the compound of formula (I) was administered by intra peritoneal *i.p.* injection at the dose of 20 mg/kg daily for 3 weeks.

### B. Results

Efficacy was evaluated *in vivo* in nude mice. A tumor reduction greater than 75% was observed.

### C. Conclusion

Therefore, the result of the tests carried out on the compounds disclosed in the present invention show that said compounds may be useful for treating and/or preventing cancer, exhibiting recruitment of MDM2 to chromatin, and more particularly but not limited to liposarcoma.

## Claims

1. A compound of formula (I), or any of its pharmaceutically acceptable salt: 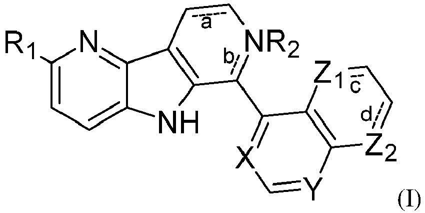 wherein:
R₁ represents a (C₁-C₆)alkyl group, a (C₃-C₆)cycloalkyl group, a (C₂-C₆)alkenyl group or a (C₁-C₆)alkoxy group, wherein one or two -CH₂- groups as present in said groups may be replaced by -O-, -S- or -NH- and wherein said groups may be substituted by one or two hydroxy group or (C₁-C₆)alkoxy group,
X and Y independently represent a -CH= group, a -CR₃= group or a -N= group,
Z1 and Z2 independently represent a -CH₂- group, a =CH- group or a =N- group,
R3 represents a hydrogen atom or a halogen atom, 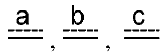 and 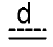 independently represent a single or double bond, and
R₂ is absent when 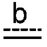 is a double bond and represents a hydrogen atom when 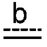 is a double
bond, and wherein when 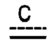 and 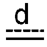 both represent a single bond, then Z1 and Z2 both represent a -CH₂- group.

2. The compound of formula (I) according to claim 1, wherein R₁ represents a (C₁-C₄)alkyl group or a (C₁-C₄)alkoxy group, wherein said groups may be substituted by one or two hydroxy group or (C₁-C₂)alkoxy group, in particular R₁ represents a (C₁-C₄)alkoxy group, and more particularly R1 represents a methoxy group.

3. The compound of formula (I) according to claim 1 or 2, wherein
- X, Y, Z₁ and Z₂ simultaneously represent a -CH= group with 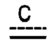 and 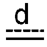 both representing a double bond,
- X and Z₂ simultaneously represent a -CH= group and Y and Z₁ represent a =N- group, with 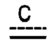 and 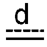 both representing a double bond,
- Y, Z₁, and Z₂ simultaneously represent a -CH= group and X represents a =N- group, with 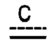 and 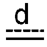 both representing a double bond,
- X, Y, and Z₂ simultaneously represent a -CH= group and Z1 represents a =N- group, with 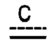 and 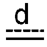 both representing a double bond,
- X, Z₁, and Z₂ simultaneously represent a -CH= group and Y represents a =N- group, with 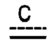 and 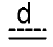 both representing a double bond,
- Y, Z₁, and Z₂ simultaneously represent a -CH= group and X represents a =N- group, with 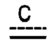 and 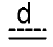 both representing a double bond,
- X, Y and Z₁ simultaneously represent a -CH= group and Z₂ represents a =N- group, with 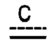 and 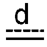 both representing a double bond, or
- X and Y simultaneously represent a -CH= group, and Z₁ and Z₂ represent a -CH₂- group, with 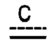 and 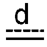 both representing a single bond.

4. The compound of formula (I) according to anyone of the preceding claims, wherein
- 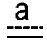 and 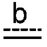 both represent a double bond and R₂ does not exist, or
- 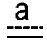 and 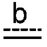 both represent a single bond and R₂ is a hydrogen atom.

5. The compound of formula (I) according to anyone of the preceding claims, or a pharmaceutically acceptable salt thereof, selected from
| No | Structure |
|---|---|
| 1 (ex 1) | 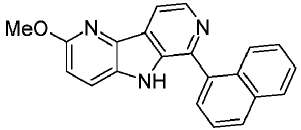 |
| 2 (ex 2) | 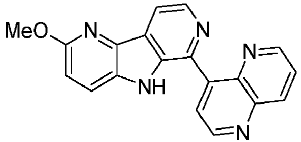 |
| 3 (ex 3) | 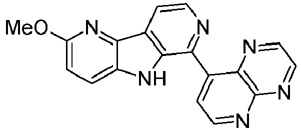 |
| 4 (ex 4) | 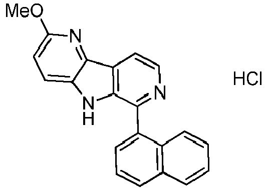 |
| 5 | 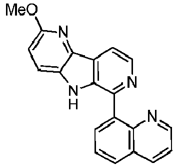 |
| 6 | 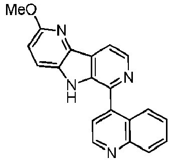 |
| 7 | 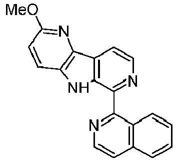 |
| 8 | 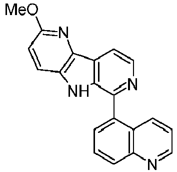 |
| 9 | 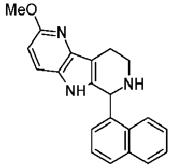 |
| 10 | 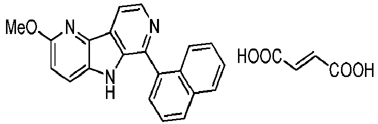 |
| 11 | 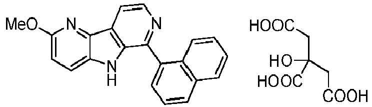 |
| 12 | 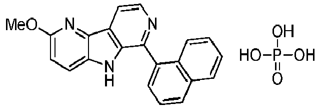 |
| 13 | 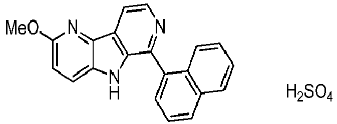 |
| 14 | 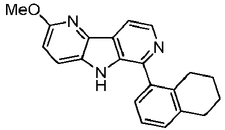 |
| 15 | 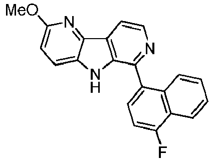 |
| 16 | 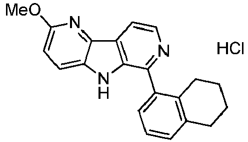 |
| 17 | 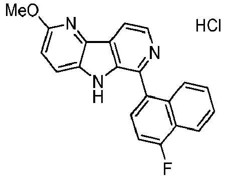 |

6. A pharmaceutical composition comprising (i) at least a compound of formula (I) as defined in anyone of the preceding claims or a pharmaceutically acceptable salt thereof, and (ii) a pharmaceutically acceptable carrier.

7. A compound of formula (I), as defined in anyone of claims 1 to 5 or a pharmaceutically acceptable salt thereof, for use as a medicament.

8. A compound of formula (I), as defined in anyone of claims 1 to 5, for use in the treatment and/or prevention of cancer, in particular cancer exhibiting recruitment of MDM2 to chromatin, more particularly selected from the group consisting of bone cancer, brain cancer, ovary cancer, breast cancer, lung cancer, colorectal cancer, osteosarcoma, skin cancer, blood cancer, including acute myeloid leukemia, pancreatic cancer, prostate cancer and liposarcoma and even more particularly selected from the group consisting of skin cancer, liposarcoma and blood cancer, for example but not limited to liposarcoma, melanoma and acute myeloid leukemia.

9. A compound of formula (I), as defined in anyone of claims 1 to 5, for use in the treatment of cancer exhibiting recruitment of MDM2 to chromatin, in particular selected from melanoma, liposarcoma and acute myeloid leukemia, in a patient who has not previously been treated by another anti-cancer drug.

10. A compound of formula (I), as defined in anyone of claims 1 to 5, for use in the treatment of a tumor, including tumor not spontaneously expressing chromatin-bound MDM2 or C-MDM2 or a tumor not exhibiting recruitment of MDM2 to chromatin before any treatment but expressing chromatin-bound MDM2 after treatment, in a patient who has been previously treated by radiotherapy, chemotherapy and/or immunotherapies.

11. Synthesis process for manufacturing a compound of formula (I) as defined in anyone of claims 1 to 5 or anyone of its pharmaceutically acceptable salts, comprising at least a step of reacting a compound of formula (II) 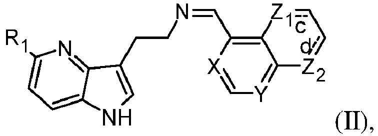 wherein R₁, X, Y, Z₁, Z₂, 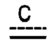 and 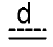 are as defined in anyone of claims 1 to 3,
under Pictet-Spengler conditions, in particular in an organic solvent, under inert atmosphere, and in presence of a catalyst, such as AlCl₃ or BF₃OEt₂, in particular under a temperature between 40 and 68°C, for between 14 and 18 hours, before cooling a room temperature, for obtaining a compound of formula (Ia), wherein 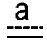 and 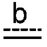 both represent single bonds and 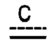 and 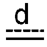 independently represent a single or double bond , which is optionally placed under aromatization conditions, in particular in an organic solvent, in presence of an heterogeneous oxidant such as potassium permanganate or copper bromide, in particular at room temperature for at least 24 hours, for obtaining a compound of formula (I), wherein 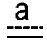 and 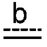 both represent double bonds and 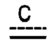 and 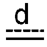 independently represent a single or double bond.

12. Synthesis process for manufacturing a compound of formula (I), wherein 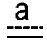 and 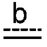 both represent a double bond and R₂ does not exist as defined in anyone of claims 1 to 4 or anyone of its pharmaceutically acceptable salts, wherein it comprises at least a step of reacting a compound of formula (III) 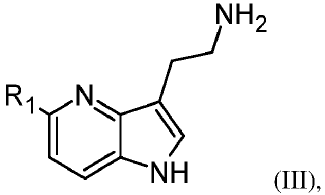 wherein R₁ is as defined in claim 1 or 2, with a compound of formula (IV) 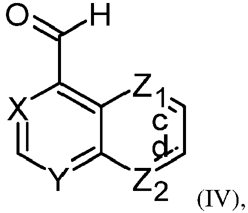 wherein, X, Y, Z₁, Z₂, 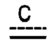 and 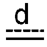 are as defined in claim 1 or 3, successively in presence of an acid, such as trifluoroacetic acid, p-toluensolfonic acid or diphenylphosphate,, under inert atmosphere, and in a second step in a solvent, such as xylene, mesitylene, EtOH or octane, in presence of a catalyst such as Pd/C, under heating to reflux, in particular for more than 8 hours, or alternatively under microwave, in an aprotic polar solvent, in presence of a catalyst, such as a mixture of ytterbium triflate and trimethysilyl chloride, ytterbium triflate, aluminium chloride, titanium chloride or other Lewis acid or Pd/C in the presence of lithium carbonate, under heat, in particular at a temperature between 100°C and 200°C.
